# EUROPEAN PATENT APPLICATION

(11) **EP 4 674 459 A1**
(43) Date of publication of application: **07.01.2026**
(21) Application number: 24844845.8
(22) Date of filing: 25.07.2024
(51) Int. Cl.: A61M 25/00, A61B 5/0215, A61M 39/10

(54) **INTERVENTIONAL MEDICAL INSTRUMENT**

(30) Priority: 27.07.2023 CN 202321991896 U
(71) Applicant: Magassist Co., Ltd., Suzhou, Jiangsu 215163 (CN)
(72) Inventor: HSU, Polin, Suzhou, Jiangsu 215163 (CN); ZHANG, Jialiang, Suzhou, Jiangsu 215163 (CN); HSU, Chiahao, Suzhou, Jiangsu 215163 (CN); WU, Tingting, Suzhou, Jiangsu 215163 (CN)
(74) Representative: Winter, Brandl - Partnerschaft mbB
(86) International application number: PCT/CN2024/107606
(87) International publication number: WO 2025/021159

(57) **Abstract**

Provided is an interventional medical device, including an interventional sheath, a catheter, and a pressure measurement assembly, wherein the pressure measurement assembly can use a first flushing liquid flowing through a gap between the interventional sheath and the catheter to measure blood pressure data of a human body, thereby allowing for monitoring of the physiological condition of a patient for interventional medical treatment. Further provided are an interventional catheter pump and a coupler thereof, applied to the interventional medical device to achieve mechanical circulatory support for a cardiac pump function of the patient. The interventional catheter pump implements pressure measurement of the first flushing liquid and a second flushing liquid by means of a first chamber and a second chamber isolated from each other and formed in the coupler, and further implements electricity supply, signal transmission, and power supply between the coupler and a driver by means of coupling connection between the coupler and the driver, thereby greatly simplifying the structure of the coupler and reducing the production difficulty and production cost.

## Description

The present application claims priority to the Chinese patent application No. 202321991896.5, titled "Interventional Medical Device", filed to the China National Intellectual Property Administration on July 27, 2023.

### Technical Field

The present application relates to the technical field of medical devices, and particularly, to an interventional medical device.

### Background

An interventional medical device is an apparatus used for disease diagnosis or minimally invasive treatment by delivering a specific device to a lesion site of a patient through a natural orifice or a minor incision by using a puncture needle, a catheter, and other interventional instruments under the monitoring of an imaging apparatus such as a digital subtraction angiography machine, a CT scanner, an ultrasound device, and a magnetic resonance imaging device. During an interventional medical treatment procedure, it is necessary to closely monitor the patient's physiological sign data such as blood pressure and heart rate. However, the current interventional medical devices lack a corresponding design.

Taking an interventional catheter pump as an example, which serves as an instance of a mechanical circulatory support (MCS) system, it is increasingly used for treating cardiac diseases such as heart failure. In the treatment of acute myocardial infarction (MI) or compensated heart failure, an interventional catheter pump can stabilize a patient after cardiogenic shock or provide support for a patient during high-risk percutaneous coronary intervention (PCI).

In the related art, an interventional catheter pump may utilize an external motor to provide power, rotational power is transmitted to a distal impeller via a driving shaft provided in a catheter, and the impeller rotates to provide flow power to blood. To understand in real time the auxiliary flow achievable during operation of a mechanical circulatory support system, the conventional techniques typically dispose a pressure sensor proximal to the impeller and estimate, using an algorithm, the flow generated by the rotation of the impeller. However, such structural design requires an additional lumen to be provided in the catheter, for a wire of a sensor to pass through. This presents significant challenges to the process and drives up production costs.

Moreover, a handle portion of the existing interventional catheter pump needs to balance multiple functions such as power generation and transmission, flushing liquid and supply thereof, electricity supply, and signal transmission, which results in an increasingly complex overall structure and poses further challenges for the design and production of the interventional catheter pump.

### Summary

To solve the problems hereinabove, the present application proposes an interventional medical device. It is possible to use the interventional medical device to set up corresponding pressure measurement assembly in the structure outside the body, instead of setting sensors for detecting blood on the structure that intervenes into the body, thereby facilitating the measurement of the blood pressure of the arterial system of a patient and simplifying the structure of an interventional portion of the device.

To achieve the objective hereinabove, the present application provides the following technical solution: an interventional medical device, including: an interventional sheath, wherein a proximal end of the interventional sheath is located outside a body of a subject and a distal end of the interventional sheath is operably inserted into the body of the subject; a catheter, wherein a distal end of the catheter is operably inserted into the body of the subject via an interior of the interventional sheath, and a gap is present between the catheter and the interventional sheath; a pressure measurement assembly, including: an operation portion, connected with a proximal end of the catheter and located outside the body of the subject, wherein a first chamber inside the operation portion that is in fluid communication with the gap; a first tubing assembly, in fluid communication with the first chamber, and configured to deliver a first flushing liquid to the first chamber; and a first pressure sensor, which is provided in the operation portion, is in fluid communication with the first chamber, and is configured to sense liquid pressure in the first chamber.

Further, the first tubing assembly includes: a first liquid storage bag, configured to store the first flushing liquid; a pressurizing device, configured to pressurize the first flushing liquid; and a control valve, configured to control the delivery of the first flushing liquid in the first liquid storage bag to the first chamber.

Further, the pressurizing device includes a fluid pump, and the fluid pump and the control valve are both provided on a tube for communicating the first liquid storage bag to the first chamber.

Further, the pressurizing device includes a pressurizing bag which includes a balloon sleeved outside the first liquid storage bag and a pressurizing pump connected with the balloon via a tube, the pressurizing pump being configured to fill the balloon with fluid to expand the balloon so as to squeeze the first liquid storage bag, thereby pressurizing the first flushing liquid.

Further, the pressurizing bag includes a pressure gauge which is provided on the tube for connecting the balloon to the pressurizing pump.

Further, the pressurizing bag includes a pressure relief valve, the pressure relief valve being provided at the balloon so as to be configured to discharge pressurized fluid from the balloon.

Further, the first tubing assembly includes a first state where the control valve is in a closed state and a second state where the control valve is in an open state, wherein liquid pressure in the first chamber in the first state is less than liquid pressure in the second state, and a time of the first tubing assembly in the first state is greater than a time of the first tubing assembly in the second state.

Further, the operation portion is further provided therein with a second chamber in fluid communication with the interior of the catheter, and the second chamber and the first chamber are sealed and isolated from each other; the interventional medical device further includes: a second tubing assembly in fluid communication with the second chamber and configured to deliver a second flushing liquid to the second chamber.

Further, the interventional medical device further includes: a second pressure sensor which is provided in the operation portion, is in fluid communication with the second chamber, and is configured to sense liquid pressure in the second chamber.

Further, the second tubing assembly includes: a second liquid storage bag, configured to store the second flushing liquid; a first liquid supply pump, configured to pump the second flushing liquid out of the second liquid storage bag; a blending device, connected with an outlet of the first liquid supply pump and in fluid communication with the second chamber via a downstream channel from the blending device to the second chamber and a return channel from the second chamber to the blending device; and a second liquid supply pump, configured to pump out the second flushing liquid from the downstream channel of the blending device.

Further, the interventional medical device further includes an action portion connected with the distal end of the catheter, the action portion being configured to perform a preset medical action.

Further, the interventional medical device includes an interventional catheter pump and including: a coupler, being at least part of the operation portion; a pump head, including a pump housing connected with the distal end of the catheter and an impeller provided inside the pump housing, wherein the impeller is driven to rotate so as to draw blood from an inlet of the pump housing and discharge the blood from an outlet of the pump housing; and a driver, detachably connected with the coupler and including a motor configured to drive the impeller.

Further, the interventional medical device includes: a circuit board, provided in the coupler and electrically connected with the first pressure sensor; a first coupling terminal, provided on the coupler and electrically connected with the circuit board; and a second coupling terminal, provided on the driver, wherein when the coupler is connected with the driver, the first coupling terminal and the second coupling terminal are coupled to each other and form an electrical connection channel.

Further, the interventional medical device further includes: a driving shaft, rotatably provided inside the catheter, wherein a distal end of the driving shaft is drivingly connected to the impeller; a driving member, driven by the motor; and a driven member, provided inside the coupler, and drivingly connected to a proximal end of the driving shaft, wherein one of the driving member and the driven member is a magnetic component, and an other of the driving member and the driven member is a magnetic component or a conductor; in a state where the coupler is connected with the driver, the driven member and the driving member are spaced apart from and coupled to each other, thereby achieving power transmission from the driving member to the driven member; the catheter extends into an interior of the coupler and is in fluid communication with the second chamber; the driven member is located inside the second chamber and the proximal end of the driving shaft extends out from the proximal end of the catheter, enters an interior of the second chamber and is drivingly connected to the driven member.

Further, the interventional sheath includes: an interventional sheath catheter; a sheath tube base, formed at a proximal end of the interventional sheath catheter, wherein the catheter is inserted from a proximal end of the sheath tube base, passes through the interventional sheath catheter, and exits from a distal end of the sheath tube base; and a side branch tube, provided on the sheath tube base and in fluid communication with an internal channel of the interventional sheath catheter, wherein an end of the side branch tube is connected with a interface of the sheath tube base to communicate with the gap between the catheter and the interventional sheath catheter, and an other end of the side branch tube is in communication with the first chamber.

Further, the sheath tube base is provided with a suture buckle to achieve fixation through the suture buckle.

Further, the coupler is provided with the first chamber, a first joint and a second joint, the first joint and the second joint are both connected with the first chamber, the first joint being in communication with a first liquid storage bag of the first tubing assembly, and the second joint being in communication with the side branch tube.

The interventional medical device provided by the present application uses the gap between the catheter and the interventional sheath as a channel communicating with the human blood circulation system and the first chamber, and provides a chamber inside the operation portion for the first flushing liquid to flow through, thereby integrating the first pressure sensor for measuring the pressure of the first flushing liquid in the operation portion, thus enabling in-vivo blood examination by disposing the sensor in vitro. Therefore, it's unnecessary to design a lumen for wiring in the catheter, thereby simplifying the structure of the catheter, reducing wire installation, and reducing the manufacturing difficulty and production costs.

### Brief Description of the Drawings

The drawings constituting part of the present application serve to provide further understanding of the present application. Illustrative embodiments of the present application and description thereof are used to explain the present application, and do not constitute improper limitation to the present application. In the drawings:
FIG. 1 is a schematic structural diagram of an interventional medical device according to an embodiment of the present application;
FIG. 2 is a schematic structural diagram of an interventional medical device according to another embodiment of the present application;
FIG. 3 is a schematic structural diagram of another embodiment of a pressurizing device of a first liquid storage bag in FIG. 1 or FIG. 2; and
FIG. 4 is a schematic diagram of an external structure of a side branch connecting coupler and an interventional sheath of an interventional catheter pump.

The drawings above include the following reference numerals:
1. Interventional sheath; 11. Interventional sheath catheter;
2. Catheter; 202. Driving shaft;
4. Pressure measurement assembly; 41. Operation portion; 411. First chamber; 42. First tubing assembly; 421. First liquid storage bag; 422. Pressurizing device; 423. Control valve; 424. Balloon; 425. Pressurizing pump; 426.Pressure gauge; 43. First pressure sensor; 44. Second chamber; 45. Second tubing assembly; 451. Second liquid storage bag; 452. First liquid supply pump; 453. Blending device; 454. Second liquid supply pump; 46. Second pressure sensor;
51. Coupler; 511. First joint; 512. Second joint; 52. Pump head; 521. Pump housing; 522. Impeller; 53. Driver;
6. Sheath tube base; 62. Suture buckle; 63. Proximal end; 64. Interface;
7. Side branch tube.

### Detailed Description of the Embodiments

Various exemplary embodiments of the present application will now be described in detail with reference to the drawings. The description of the exemplary embodiments is merely illustrative and is not intended to limit the present application and application or use thereof in any way. The present application is implemented in many different forms and is not limited to the embodiments herein. These embodiments are provided to make the present application thorough and complete, and to fully convey the scope of the present application to a person skilled in the art. It should be noted that: unless otherwise specifically stated, the relative disposition of components and steps, the composition of materials, numerical expressions, and numerical values set forth in these embodiments should be interpreted as merely examples and not as limitations.

The terms "proximal" and "distal" as used in this embodiment are relative to a clinician who operates the device. The term "proximal" refers to a portion close to the clinician, while "distal" refers to a portion far away from the clinician. A proximal end of a certain component/assembly refers to the end close to the clinician, while a distal end refers to the end far away from the clinician.

During interventional examination or treatment, an interventional sheath and a catheter are often used in combination. The interventional sheath primarily functions to enlarge a percutaneous incision and assist the catheter in entering the vasculature of a patient. During clinical use, first a small incision is made on the skin of a puncture site by means of a scalpel, and then the puncture site is punctured by means of a puncture needle and a guidewire is threaded in along a needle tube. Subsequently the interventional sheath with a dilator inserted therein is threaded through the tail end of the guidewire and advanced to the blood vessel. Finally, the dilator is withdrawn. Thus, a percutaneous access pathway is established for an intravascular device by delivering the front end of the interventional sheath to a first desired position in the patient's body.

On the basis of a percutaneous access pathway for the intravascular device established by the interventional sheath, the catheter is threaded through the interior of the interventional sheath and delivered into a human blood vessel and reaches a second desired location in the patient's body under the guidance of the guidewire.

Depending on different purposes of interventional examination or treatment, the catheter has different functions accordingly.

For example, during interventional examination, a contrast agent is delivered to a specific position in the body via the catheter for angiographic imaging diagnosis, or a local lesion is punctured via the catheter to extract a tissue for pathological diagnosis.

For another example, during interventional treatment, a drug or an embolic agent is injected into a lesion area in the body via the catheter for treatment, or a device such as a stent is placed at a designated position in the body via the catheter, or a driving shaft is disposed via the catheter to provide power for a human blood pump so as to assist the pumping function of the heart.

In different purposes, an action portion as described below is generally also different. The action portion is changeable on the basis of different scenarios to achieve the purpose of corresponding to the scenarios. For example, during interventional treatment, the action portion is a pump head with blood-pumping function as described below, or a vascular stent capable of dilating the blood vessel, etc.

For multiple interventional examinations or treatments introduced above, the interventional sheath and the catheter constitute the structural foundation for achieving a specific medical purpose. Since the catheter needs to pass through the interior of the interventional sheath to percutaneously enter the human body, there is often a certain gap between the interventional sheath and the catheter. Moreover, since the distal ends of the interventional sheath and the catheter extend into the blood circulation system of the human body, blood is prone to enter the gap between the interventional sheath and the catheter, thereby increasing the risk of thrombosis. The hazards of thrombosis are evident, including, but not limited to, thrombi flowing with the bloodstream throughout the body and entering the brain to cause stroke, or thrombi remaining stationary in blood vessel leading to inadequate oxygen supply to nearby neural tissues and resulting in nerve damage, among others.

Meanwhile, during interventional examination or treatment, a physician also desires to monitor the vital sign data of the subject in real time, particularly blood pressure index that can characterize the operational status of the blood circulation system. In particular, in a mechanical circulatory support system exemplified by an interventional catheter pump, it is desirable to know in real time the achievable auxiliary flow rate when an impeller is operating. One means is to calculate it via an algorithm by measuring the blood pressure in the aorta, and thus it is crucial to accurately obtain the aortic blood pressure.

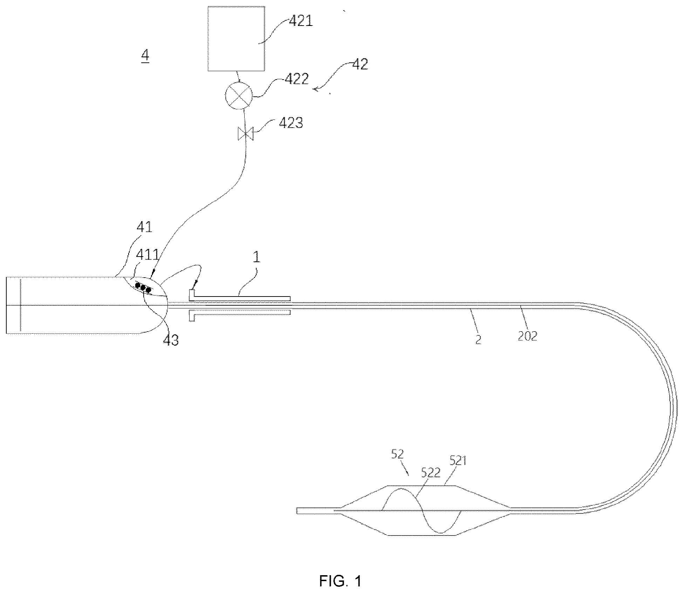

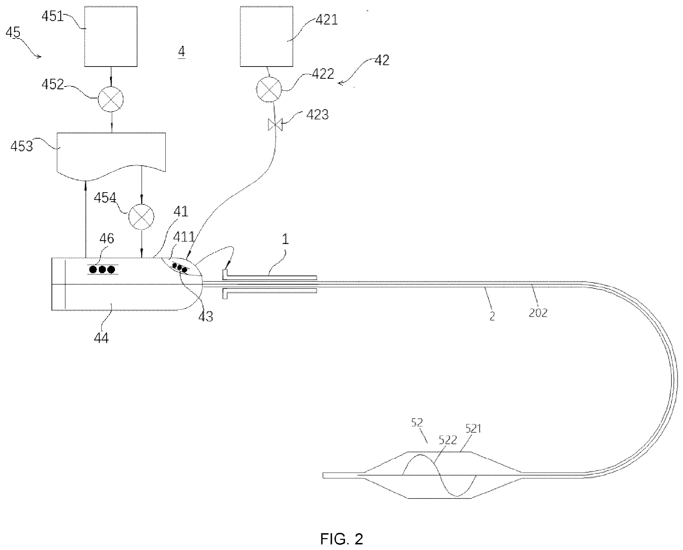

On the basis of the structural foundation of the interventional sheath and the catheter hereinabove and the need to accurately obtain the aortic blood pressure, as shown in FIGs. 1 and 2, the present embodiment provides an interventional medical device including a pressure measurement assembly. The interventional medical device performs a preset medical action via an action portion connected with a distal end of a catheter 2 and the blood pressure of the patient is measured in vitro by means of the pressure measurement assembly 4 via a gap between the interventional sheath 1 and the catheter 2.

The pressure measurement assembly 4 includes an operation portion 41 connected with a proximal end of the catheter 2, wherein the operation portion 41 is located outside the body of a subject and is provided therein with a first chamber 411 in fluid communication with the gap between the interventional sheath 1 and the catheter 2. On the basis of the first chamber 411, the pressure measurement assembly 4 further includes a first tubing assembly 42 in fluid communication with the first chamber 411, wherein the first tubing assembly 42 is configured to deliver a first flushing liquid to the first chamber 411. Thus, the first flushing liquid enters the first chamber 411 via the first tubing assembly 42, and is used to flush the gap between the catheter 2 and the interventional sheath 1, thereby effectively preventing thrombus formation. On this basis, in the present application, a first pressure sensor 43 is provided in the operation portion 41, and configured to sense the liquid pressure in the first chamber 411, thereby converting the pressure of the first flushing liquid in the first chamber 411 to obtain blood pressure data of the human body.

The principle of converting the pressure of the first flushing liquid into the blood pressure data of the human body hereinabove is that: in a closed tubing system, the pressures at various intercommunicated positions of the tube system are equal. In particular, for the interventional sheath 1 and the catheter 2 extending into the human body, their distal ends extend into the blood circulation system while their proximal ends are in fluid communication with the first chamber 411 in the operation portion 41. Therefore, the first chamber 411, the gap between the interventional sheath 1 and the catheter 2, and the blood circulation system in the human body jointly form an intercommunicated closed tubing system. Thus, it can be obtained the blood pressure data of the human body by sensing the liquid pressure in the first chamber 411 by means of the first pressure sensor 43.

Since the first pressure sensor 43 is provided inside the extracorporeal operation portion 41 in the present application, it eliminates the need for installing and deploying a wire in the catheter 2 compared to the technical solution where the pressure sensor is provided inside the body. Therefore, the structural complexity, process difficulty, and manufacturing cost of the pressure measurement system are significantly reduced.

Furthermore, clinical practice has also demonstrated that there is no need to deploy a wire connected with the sensor in the catheter 2, such that the diameter of the catheter 2 can be significantly reduced, thereby enabling the reduction of the interventional size of the device. Moreover, the reduction in the diameter of the catheter 2 implies that the diameter of the interventional sheath 1 matched therewith can also be reduced accordingly. Thus, during the working process after the intervention of the action portion is completed, it is unnecessary to maintain the puncture site of the patient in a greatly dilated state. The small interventional size and puncture site will not be excessively dilated for a prolonged period, which is greatly beneficial for reducing patient's suffering, avoiding complications, and expediting postoperative recovery of the patient.

The first pressure sensor 43 is provided in the first chamber 411, such that the blood pressure can be measured at the position where the interventional sheath 1 and the catheter 2 extend into the distal end of the blood circulation system by means of the gap between the interventional sheath 1 and the catheter 2, making the blood pressure measurement position closer to a target area of interventional examination or treatment. Thus, compared with non-interventional in-vitro blood pressure measurement, the blood pressure measurement result is closer to the true blood pressure condition of the target area, thereby providing more accurate and detailed blood pressure data for interventional examination or treatment.

The first tubing assembly 42 includes a first liquid storage bag 421, a pressurizing device 422, and a control valve 423. The first liquid storage bag 421 is configured to store the first flushing liquid. The control valve 423 is configured to control in real time the on/off of the first flushing liquid to the first chamber 411 and is further configured to adjust the supply flow rate of the first flushing liquid from the first liquid storage bag 421 to the first chamber 411. The pressurizing device 422 acts on the first liquid storage bag 421 and is configured to pressurize the first flushing liquid in the first liquid storage bag 421 to a set pressure value, so as to meet the pressure requirements of the human blood circulation system and the gap between the interventional sheath 1 and the catheter 2 for the first flushing liquid.

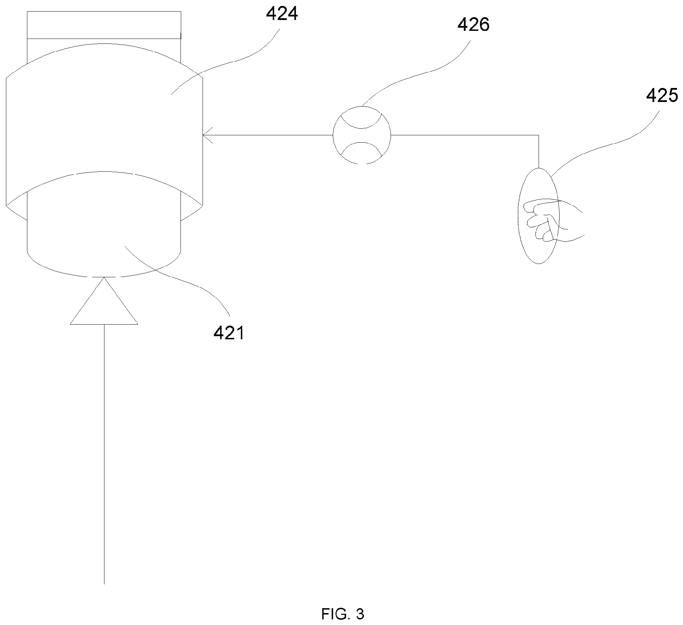

The pressurizing device 422 is any structure capable of providing a pressurizing effect for the first flushing liquid. For example, in an optional embodiment, the pressurizing device 422 is a fluid pump as shown in FIG. 1 or 2, such as a peristaltic pump, which is provided on a tube for communicating the first liquid storage bag 421 to the first chamber 411. Alternatively, in another optional embodiment, the pressurizing device 422 is a pressurizing bag as shown in FIG. 3, including a balloon 424 sleeved outside the first liquid storage bag 421 and a pressurizing pump 425 connected with the balloon via a tube. The pressurizing pump 425 is a manual pressurizing pump or an electric pressurizing pump, configured to fill the balloon 424 with fluid to expand the balloon 424 so as to squeeze the first liquid storage bag 421, thereby pressurizing the first flushing liquid. A pressure gauge 426 is provided on the tube for connecting the balloon to the pressurizing pump, so as to facilitate real-time observation of a pressurizing value. The balloon 424 is provided with a pressure relief valve so as to be configured to discharge pressurized fluid (e.g., gas) from the balloon to achieve pressure relief of the pressurizing bag. The control valve 423 is provided on a tube for communicating the first liquid storage bag 421 to the first chamber 411, and can have any suitable existing configuration, such as a solenoid valve, a tube clamp, or the like.

For the first tubing assembly 42 of the present embodiment, it is not only necessary to realize the transport and pumping function of the first flushing liquid, but also necessary to assist the first pressure sensor 43 in realizing its pressure sensing function. It should be understood that if the pressure is measured during pumping of the first flushing liquid, the pressure measurement value will be closer to the output pressure of the pressurizing device 422 of the first tubing assembly 42 than to the human blood pressure. Therefore, in the present application, the state where the first tubing assembly 42 delivers the first flushing liquid to the first chamber 411 is configured to at least include a first state and a second state.In particular, the pressure of the first flushing liquid in the first state is less than the pressure in the second state. Moreover, the pressure of the flushing liquid in the first state when the pressure is stabilized is substantially equal to the human blood pressure, while the pressure of the flushing liquid in the second state is greater than the human blood pressure. As such, the first flushing liquid is enabled to pass through the gap between the interventional sheath 1 and the catheter 2 at a higher pressure value and enter the human blood system, thereby fully flushing the gap between the interventional sheath 1 and the catheter 2 to flush out the blood permeating into the gap, and reducing or avoiding thrombus formation.

On the basis of the technical description hereinabove, it can be understood that the first state is a state where the control valve 423 is in a closed state and the second state is a state where the control valve 423 is in an open state. When the control valve 423 is opened, since the pressure of the flushing liquid is greater than the human blood pressure at this time, the first flushing liquid rapidly flows out through the gap between the interventional sheath 1 and the catheter 2, thereby flushing out the blood retained in the gap and preventing the blood in the gap from remaining for a prolonged period to form a thrombus. After flushing for a predetermined duration, the control valve 423 is closed. At the moment when the control valve 423 is closed, the pressure of the flushing liquid in the gap remains greater than the blood pressure, thereby causing the flushing liquid in the gap to continue flowing out from the distal end. As the flushing liquid flows out, the pressure of the flushing liquid in the gap gradually decreases until it becomes substantially equal to the blood pressure, at which point the outflow stops and a pressure-stable state is reached. Since the first chamber 411 is in fluid communication with the human blood circulation system through the gap, the pressure in the first chamber 411 as measured by the first pressure sensor 43 is substantially the human blood pressure.

The pressurizing device 422 and the control valve 423 further control the duration of the first flushing liquid in the first state to be greater than the duration in the second state. It should be understood that during interventional examination or treatment, although the first flushing liquid is heparinized saline or other similar liquids to reduce the impact on the blood circulation system when the first flushing liquid enters the human body. However, it should still be avoided that an excessive amount of the first flushing liquid enters the human body, so as to prevent disrupting the original equilibrium state of the human blood circulation system. Therefore, the flushing method for the gap between the interventional sheath 1 and the catheter 2 is intermittent rather than continuous. For example, in an embodiment, after the control valve 423 is opened for flushing for a predetermined duration of 2 to 3 seconds (or even shorter), the control valve 423 is rapidly closed.

For the embodiment hereinabove, the first state is regarded as a normal state of the first tubing assembly 42, and the second state is regarded as a flushing state of the first tubing assembly 42. However, it should be noted that with development of technology and requirement for actual examination or treatment, for the interventional medical device, the first state hereinabove is further divided into a plurality of more specific states. In this regard, the magnitude relationships of the time and pressure defined in the present application are no longer limited to the first state and the second state in the embodiment hereinabove.

It should be understood that the present embodiment defines that the liquid pressure in the second state is greater than the liquid pressure in the first state to ensure that the first flushing liquid has sufficient kinetic energy to flush the gap between the interventional sheath 1 and the catheter 2, and defines that the a time of the flushing liquid in the second state is shorter than the a time in the first state to reduce excessive entry of the first flushing liquid into the blood circulation system which can affect human homeostasis. On the basis of this concept, a person skilled in the art can adjust the liquid pressure and the time in the first state and the second state depending on actual needs, and the present embodiment imposes no additional limitations thereto.

As shown in FIG. 2, in another embodiment, the operation portion 41 is further provided with a second chamber 44 therein, wherein the second chamber 44 and the first chamber 411 are sealed and isolated from each other and are not in communication with each other. A second tubing assembly 45 is in fluid communication with the second chamber 44 and is configured to deliver a second flushing liquid to the second chamber 44. The pressure measurement system further includes a second pressure sensor 46 which is also provided in the operation portion 41 and configured to sense the pressure of the second chamber 44, thereby assisting in obtaining real-time perfusion pressure of the second flushing liquid and providing assistance for interventional examination or treatment.

The second chamber 44 and the second tubing assembly 45 cooperating therewith are configured to pump the second flushing liquid into the catheter 2, and the second flushing liquid flows distally along the catheter 2, thereby lubricating and cooling rotating components in the catheter 2 and rotating components of the action portion. Particularly, when the solution of the present embodiment is applied to an interventional catheter pump scenario, the rotating component in the catheter 2 is a flexible driving shaft 202, and the rotating component of the action portion is a proximal bearing and a distal bearing that support rotation of the impeller 522. Regarding the solution where the second chamber 44 cooperates with the second tubing assembly 45 to achieve the lubrication and cooling hereinabove, reference can made to the description in Chinese patent ZL202211578294.7, which will not be repeated herein.

The first pressure sensor 43 and the second pressure sensor 46 are both provided in the operation portion 41. By means of the first chamber 411 and the second chamber 44 which are separated from and are not in fluid communication with each other in the operation portion 41, the first pressure sensor 43 can sense human blood pressure on the basis of the first chamber 411, and the second pressure sensor 46 can sense the pressure of the second flushing liquid on the basis of the second chamber 44. Thus, the pressure measurement system of the present application is capable of accurately sensing the pressure of extracorporeal liquids and intracorporeal blood involved in interventional examination or treatment, improving the acquisition timeliness and accuracy of the pressure data during interventional examination or treatment. Additionally, by providing both the first pressure sensor 43 and the second pressure sensor 46 in the operation portion 41, the integration level of the pressure measurement system is further improved, and the structural design is reasonable.

The second tubing assembly 45 includes a second liquid storage bag 451, a first liquid supply pump 452, a blending device 453 and a second liquid supply pump 454. The second liquid storage bag 451 stores the second flushing liquid, and the first liquid supply pump 452 is a peristaltic pump, is provided on a tube connecting the second liquid storage bag 451 to the blending device 453, and is configured to continuously pump out of the second flushing liquid from the second liquid storage bag 451. The blending device 453 is connected with an outlet of the first liquid supply pump 452 and is provided with a downstream channel from the blending device 453 to the second chamber 44 and a return channel from the second chamber 44 to the blending device 453, and the second liquid supply pump 454 can also be a peristaltic pump and is configured to pump out of the second flushing liquid from the downstream channel of the blending device 453.

Under the action of the first liquid supply pump 452 and the second liquid supply pump 454, the second flushing liquid is pumped out from the second liquid storage bag 451 and supplied to the second chamber 44 via the downstream channel of the blending device 453. To ensure that the flow rate of the second flushing liquid in the second chamber 44 for perfusion into the human body meets the demand, a return channel is further provided between the blending device 453 and the second chamber 44, whereby part of the second flushing liquid pumped into the second chamber 44 will enter the human body through the catheter 2, while the remaining part will return to the blending device 453 via the return channel. Since the flow rate of the second flushing liquid through the catheter 2 will depend on the flow area of the catheter 2, the provision of the return channel can ensure that the liquid flow rate entering the human body through the second chamber 44 does not become excessive.

As shown in FIGs. 1, 2, and 4, in a specific embodiment, the interventional medical device is an interventional catheter pump which includes a coupler 51, a pump head 52, and a driver 53. The pump head 52 includes a pump housing 521 connected with the distal end of the catheter 2 and an impeller 522 provided inside the pump housing 521, wherein the impeller 522 is driven to rotate so as to draw blood from an inlet of the pump housing 521 and discharge the blood from an outlet of the pump housing 521. The coupler 51 is detachably coupled to the driver 53, and the coupling connection therebetween enables transmission of power from the driver 53 to the coupler 51, which, in turn, drives the impeller 522 to rotate.

Similarly, regarding the relevant structure of the interventional catheter pump and the non-contact coupling method between the coupler 51 and the driver 53, reference can also be made to the description in Chinese patent No. ZL202211578294.7, which will not be repeated herein.

Thus, the coupler 51 of the interventional catheter pump can serve as at least part of the operation portion 41 of the interventional medical device, while the pump head 52 can serve as at least part of a working portion of the interventional medical device, thereby utilizing the blood flow driven by the rotation of the impeller 522 to support the pumping function of the heart of the patient. It should be understood that the interventional medical device can perform multiple different therapeutic or diagnostic actions according to patient needs, for example, "simultaneously" achieving drug injection and stent placement for the patient, or "simultaneously" achieving angiographic diagnosis and mechanical circulatory support for the patient. In this case, the operation portion 41 and the working portion of the interventional medical device can each include operation components and working components corresponding to different purposes, and different medical operations can be completed by the interventional medical device constructed by the same interventional sheath 1, catheter 2 and pressure measurement assembly 4, thereby saving the material cost of interventional medical treatment, reducing medical treatment time and alleviating patient's suffering.

It should be noted that the "simultaneously" hereinabove does not define different types of interventional medical treatments to be performed at the same time, but rather means that the interventional medical device is constructed by the same interventional sheath 1, catheter 2 and pressure measurement assembly 4. Of course, different types of interventional medical treatments can also be performed on a patient at the same time, in which case the pressure measurement assembly 4 provided by the present application can still accurately and timely obtain the blood pressure data of the patient's body, thereby assisting the physician in monitoring the physiological condition of the patient and make medical decisions.

The coupler 51 is provided with a circuit board therein. A first coupling terminal is provided at a proximal end of the coupler 51, and a second coupling terminal is provided at a distal end of the driver 53. When the coupler 51 is connected with the driver 53, the first coupling terminal and the second coupling terminal are connected with each other to form an electrical connection channel. The circuit board is electrically connected with the first pressure sensor 43 and the second pressure sensor 46 on one hand, and electrically connected with the first coupling terminal on the other hand. Thus, an electrical connection path from the first pressure sensor 43 and the second pressure sensor 46 to the circuit board and then to the first coupling terminal is formed in the coupler 51, and an electrical connection path from the first coupling terminal to the second coupling terminal is formed outside the coupler 51.

When the coupler 51 is connected with the driver 53, an electrical connection channel is formed by connecting the first coupling terminal to the second coupling terminal. Thus, in the present embodiment, the electrical connection path inside the coupler 51 is connected into the driver 53, such that the driver 53 is used to provide electrical energy to the coupler 51, avoiding adverse effects on the electrical connection channel caused by leakage of the first flushing liquid or the second flushing liquid in the coupler 51.

An electrical connection channel between the circuit board and the first pressure sensor 43, the second pressure sensor 46 and the first coupling terminal, as well as the electrical connection channel formed between the first coupling terminal and the second coupling terminal, can not only transmit electrical energy for powering electronic members but also transmit corresponding sensing signals or control signals. That is, the first pressure sensor 43 and the second pressure sensor 46 can transmit pressure signals sensed in the first chamber 411 and the second chamber 44 to the circuit board through the electrical connection path, and then to the driver 53 through the first coupling terminal and the second coupling terminal. The wired communication formed by the electrical connection path can effectively improve the reliability and real-time performance of communication, which is crucial for ensuring the safe operation of the interventional medical device and the life health of the patient.

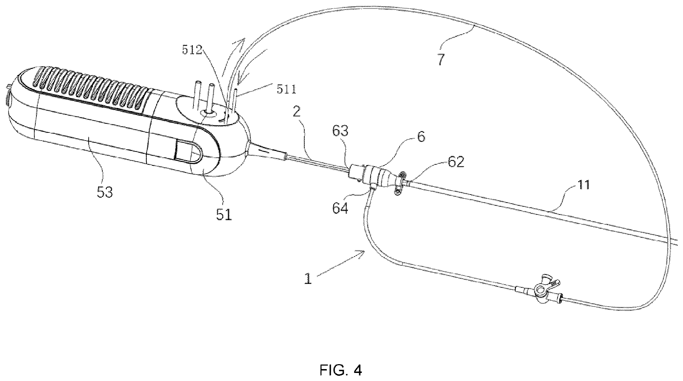

As shown in FIG. 4, the coupler 51 is provided with a first joint 511 and a second joint 512 in communication with the first chamber 411, wherein the first joint 511 is communicated with the first liquid storage bag 421 via a tube, and the second joint 512 is communicated with the gap between the interventional sheath 1 and the catheter 2 via a side branch tube 7. On the basis of the surrounding position relationship of the interventional sheath 1 relative to the catheter 2, the first tubing assembly 42 supplies liquid from the first liquid storage bag 421 to the first chamber 411 along a liquid inflow direction as shown in the figure, then the first flushing liquid in the first chamber 411 enters the side branch tube 7 of the interventional sheath 1 along a liquid outflow direction as shown in the figure, and ultimately flows to the first desired position via the gap between the interventional sheath 1 and the catheter 2, and the second flushing liquid in the second chamber 44 flows to the second desired position through the catheter 2.

The first desired position varies depending on the specific application scenario of the catheter pump. For example, in a scenario where the catheter pump serves for left ventricular assistance, the first desired position is a certain position located in the left ventricle, and the second desired position is a certain position located in the aorta. In a scenario where the catheter pump serves for right ventricular assistance, the first desired position is approximately a certain location in the pulmonary vein, and the second desired position is a certain position in the right ventricle.

As shown in FIGs. 1 and 2, the catheter 2 is provided therein with a driving shaft 202, wherein the driving shaft 202 is preferably a flexible driving shaft which can bend along with the catheter 2 in the vascular system of the human body so as to extend into a desired position in the human body. The pump housing 521 is connected with the distal end of the catheter 2, and the impeller 522 is provided inside the pump housing 521. The pump housing 521 is deployed upon reaching a desired position in the human body such as the left ventricle, and the impeller 522 provided therein is driven by rotational power provided by the driving shaft 202 to draw blood at the left ventricle from the inlet of the pump housing 521 and discharge it at the aorta from the outlet of the pump housing 521, thereby achieving an auxiliary effect on the cardiac pump function.

The catheter pump hereinabove is a foldable catheter pump, and the pump housing 521 together with the impeller 522 are both foldable. It should be noted that the application scenarios of the present embodiment are not limited thereto. In fact, the solution of the present application is also applicable to a non-foldable catheter pump. Similarly, the catheter pump hereinabove is an external power device. On the basis of the description above, the catheter pump can also adopt a built-in power device structure. At this time, a power device such as a motor is connected with the distal end of the catheter 2 which no longer has an elongate flexible driving shaft 202 provided therethrough, and the power device drives the impeller 522 by means of a short rigid shaft or magnetic coupling, etc.

In the interventional catheter pump, the first flushing liquid enters the first desired position in the human body through the gap between the catheter 2 and the interventional sheath 1, thereby achieving a flushing effect on the gap between the catheter 2 and the interventional sheath 1 and preventing thrombus formation. Moreover, since the flushing of the gap between the catheter 2 and the interventional sheath 1 by the first flushing liquid is intermittent, during the flushing process by the first flushing liquid, the first pressure sensor 43 can sense the pressure of the first flushing liquid, and in addition to the flushing by the first flushing liquid, by means of the equilibrium established between the pressure of the first flushing liquid in the gap between the catheter 2 and the interventional sheath 1 and the blood pressure of the human body, the first pressure sensor 43 can sense the blood pressure of the human body. In a scenario where the catheter pump serves for left ventricular assistance, the blood pressure hereinabove particularly refers to the blood pressure in the aorta. Moreover, since a blood outlet of the pump head 52 is located in the aorta, the blood pressure hereinabove substantially reflects the pumping pressure applied by the pump head 52 to the blood.

The second flushing liquid flows from the catheter 2 into the second desired position in the human body, thereby achieving lubrication and cooling effects on the catheter 2, the rotating driving shaft therein, as well as the proximal and distal bearings. In this regard, the second pressure sensor 46 can sense in real time the pressure of the second flushing liquid.

The interventional catheter pump further includes a driving member and a driven member, wherein the driving member is driven by the motor, and the driven member is provided inside the coupler 51, and is drivingly connected to the proximal end of the driving shaft. In a state where the coupler 51 is connected with the power device, the driven member and the driving member are spaced apart and coupled to each other, thereby achieving power transmission from the driving member to the driven member, and further, by means of the transmission connection between the driven member and the driving shaft 202, the power of the motor is transmitted through the driving member, the driven member, and the driving shaft 202 to the impeller 522 for rotation of the impeller 522 to pump blood.

The driving member and the driven member adopt non-contact transmission, which can effectively prevent the second flushing liquid from intruding into the interior of the driver 53 and affecting electrical members such as the motor. In particular, the catheter 2 extends into the interior of the coupler 51 and is in fluid communication with the second chamber 44. The driven member is located inside the second chamber 44. The proximal end of the driving shaft protrudes from the proximal end of the catheter 2, enters the interior of the second chamber 44 and is drivingly connected to the driven member, such that the driven member is drivingly connected to the driving shaft inside the second chamber 44, thus further confining the second flushing liquid within the second chamber 44 of the coupler 51 and the catheter 2, and preventing the second flushing liquid from flowing into other parts of the coupler 51 and causing adverse effects.

The interventional sheath 1 can adopt any suitable existing configuration, for example, as provided in the known embodiment disclosed in the patent publication No. CN115227962A. As shown in FIG. 4, the interventional sheath 1 generally includes an elongated interventional sheath catheter 11, a sheath tube base 6 formed at a proximal end of the interventional sheath catheter 11, and a side branch tube 7 provided on the sheath tube base 6 and in fluid communication with an internal channel of the interventional sheath catheter 11. The catheter 2 is threaded in through a proximal end 63 of the sheath tube base 6, further passes through the interventional sheath catheter 11, and exits from a distal end of sheath tube base 6. One end of the side branch tube 7 is connected with the sheath tube base 6 via a interface 64, and further communicates with the gap hereinabove formed between the interventional sheath and the catheter after the catheter 2 is threaded into the interventional sheath, while the other end of the side branch tube 7 communicates with the first chamber 411. During the entire operation of the pump head 52, the interventional sheath 1 is fixed to the patient's skin by means of a suture buckle 62 provided on the sheath tube base 6, thereby achieving fixation.

It should be noted that, in order to achieve sealing and prevent liquid leakage and blood seepage, the sheath tube base 6 is provided with a sealing structure therein. For details, reference can made to the known embodiments hereinabove, which will not be repeated herein.

The coupler 51 is connected with the catheter 2, thereby serving as an intermediate structure to provide the second flushing liquid for the catheter 2, provide the first flushing liquid for the gap between the catheter 2 and the interventional sheath 1, and provide measurement data of the first flushing liquid and blood pressure by means of the gap between the catheter 2 and the interventional sheath 1. Moreover, the coupler 51 is matched and connected with the driver 53, thereby achieving coupling connections of electrical energy, communication signals, and rotational driving.

The coupler 51 includes a housing, and the first chamber 411 and the second chamber 44 that are provided in the housing. The first chamber 411 is configured to receive the first flushing liquid and deliver the first flushing liquid to the gap between the catheter 2 and the interventional sheath 1. The second chamber 44 is separated from the first chamber 411 and is configured to receive the second flushing liquid and deliver the second flushing liquid into the catheter 2.

On the basis of the structure of the mutually isolated first chamber 411 and second chamber 44 in the coupler 51, the first pressure sensor 43 and the second pressure sensor 46 are provided inside the housing to measure the pressures of the first chamber 411 and the second chamber 44, respectively.

As described above, the first flushing liquid in the first chamber 411 has at least two states. Therefore, the first pressure sensor 43 measures first flushing liquid pressure of the first flushing liquid in the flushing state, and measures the blood pressure in a non-flushing static state. The second pressure sensor 46 can obtain perfusion pressure of the second flushing liquid into the body by measuring the pressure of the second flushing liquid in the second chamber 44.

The coupling method between the driving member and the driven member is magnetic coupling, in which case both the driving member and the driven member are magnetic components. Alternatively, the coupling method between the driving member and the driven member is an eddy current coupling, in which case both the driving member and the driven member are a magnetic component and a conductor, respectively.

On the basis of the coupling connection between the first coupling terminal and the second coupling terminal, and the coupling connection between the driven member and the driving member, the electricity supply, signal transmission, and power supply inside the coupler 51 can all be provided by a power device external to the coupler 51 in a non-contact manner, thereby forming a relatively independent sealed space in the coupler 51 to avoid adverse effects on the electricity supply, signal transmission, and power supply caused by leakage of the first flushing liquid and the second flushing liquid.

The catheter 2 extends into the coupler 51 and is in fluid communication with the second chamber 44. The driven member is located inside the second chamber 44. The proximal end of the driving shaft extends from the catheter 2 into the second chamber 44 and is drivingly connected to the driven member. On this basis, the driven member is transmissibly connected, inside the second chamber 44, to the connecting shaft, thereby further confining the second flushing liquid inside the second chamber 44 of the coupler 51 and the catheter 2, and avoiding adverse effects due to the second flushing liquid flowing to other parts of the coupler 51.

In summary, the interventional medical device provided by the present application can utilize the gap between the catheter 2 and the interventional sheath 1 as well as the two states of the first flushing liquid in the gap. In the flushing state of the first flushing liquid, the gap between the catheter 2 and the interventional sheath 1 is flushed to prevent thrombus formation and in the non-flushing state of the first flushing liquid, the gap between the catheter 2 and the interventional sheath 1 serves as a tube communicated with the human blood circulation system and the first chamber 411 to accurately measure the human blood pressure.

Furthermore, in the present application, a chamber for the first flushing liquid to flow through is provided in the coupler 51 of the interventional catheter pump, such that the first pressure sensor 43 for measuring the pressure of the first flushing liquid is integrated in the coupler 51. This configuration simplifies the structure of the catheter 2 and the installation of the wire by placing the sensor in vitro, reducing the manufacturing difficulty and production costs.

Although the embodiments disclosed in the present application are as described above, the described only involves the embodiments which are used in order to facilitate understanding of the present application and is not intended to limit the present application. Any person skilled in the art to which the present application pertains can make any modifications and variations in the form and details of implementation without departing from the spirit and scope disclosed in the present application.

## Claims

1. An interventional medical device, comprising:
an interventional sheath, wherein a proximal end of the interventional sheath is located outside a body of a subject and a distal end of the interventional sheath is operably inserted into the body of the subject;
a catheter, wherein a distal end of the catheter is operably inserted into the body of the subject via an interior of the interventional sheath, and a gap is present between the catheter and the interventional sheath;
a pressure measurement assembly, comprising:
an operation portion, connected with a proximal end of the catheter and located outside the body of the subject, wherein a first chamber inside the operation portion that is in fluid communication with the gap;
a first tubing assembly, in fluid communication with the first chamber, and configured to deliver a first flushing liquid to the first chamber; and
a first pressure sensor, which is provided in the operation portion, is in fluid communication with the first chamber, and is configured to sense liquid pressure in the first chamber.

2. The interventional medical device according to claim 1, wherein the first tubing assembly comprises:
a first liquid storage bag, configured to store the first flushing liquid;
a pressurizing device, configured to pressurize the first flushing liquid; and
a control valve, configured to control the delivery of the first flushing liquid in the first liquid storage bag to the first chamber.

3. The interventional medical device according to claim 2, wherein the pressurizing device comprises a fluid pump, and the fluid pump and the control valve are both provided on a tube for communicating the first liquid storage bag to the first chamber.

4. The interventional medical device according to claim 2, wherein the pressurizing device comprises a pressurizing bag which comprises a balloon sleeved outside the first liquid storage bag and a pressurizing pump connected with the balloon via a tube, the pressurizing pump being configured to fill the balloon with fluid to expand the balloon so as to squeeze the first liquid storage bag, thereby pressurizing the first flushing liquid.

5. The interventional medical device according to claim 4, wherein the pressurizing bag comprises a pressure gauge which is provided on the tube for connecting the balloon to the pressurizing pump.

6. The interventional medical device according to claim 4, wherein the pressurizing bag comprises a pressure relief valve, the pressure relief valve being provided at the balloon so as to be configured to discharge pressurized fluid from the balloon.

7. The interventional medical device according to claim 2, wherein the first tubing assembly comprises a first state where the control valve is in a closed state and a second state where the control valve is in an open state, wherein liquid pressure in the first chamber in the first state is less than liquid pressure in the second state, and a time of the first tubing assembly in the first state is greater than a time of the first tubing assembly in the second state.

8. The interventional medical device according to claim 1, wherein the operation portion is further provided therein with a second chamber in fluid communication with the interior of the catheter, and the second chamber and the first chamber are sealed and isolated from each other; the interventional medical device further comprises: a second tubing assembly in fluid communication with the second chamber and configured to deliver a second flushing liquid to the second chamber.

9. The interventional medical device according to claim 8, wherein the interventional medical device further comprises: a second pressure sensor which is provided in the operation portion, is in fluid communication with the second chamber, and is configured to sense liquid pressure in the second chamber.

10. The interventional medical device according to claim 8, wherein the second tubing assembly comprises:
a second liquid storage bag, configured to store the second flushing liquid;
a first liquid supply pump, configured to pump the second flushing liquid out of the second liquid storage bag;
a blending device, connected with an outlet of the first liquid supply pump and in fluid communication with the second chamber via a downstream channel from the blending device to the second chamber and a return channel from the second chamber to the blending device; and
a second liquid supply pump, configured to pump out the second flushing liquid from the downstream channel of the blending device.

11. The interventional medical device according to claim 10, further comprising an action portion connected with the distal end of the catheter, the action portion being configured to perform a preset medical action.

12. The interventional medical device according to claim 10, comprising an interventional catheter pump and comprising:
a coupler, being at least part of the operation portion;
a pump head, comprising a pump housing connected with the distal end of the catheter and an impeller provided inside the pump housing, wherein the impeller is driven to rotate so as to draw blood from an inlet of the pump housing and discharge the blood from an outlet of the pump housing; and
a driver, detachably connected with the coupler and comprising a motor configured to drive the impeller.

13. The interventional medical device according to claim 12, comprising:
a circuit board, provided in the coupler and electrically connected with the first pressure sensor;
a first coupling terminal, provided on the coupler and electrically connected with the circuit board; and
a second coupling terminal, provided on the driver,
wherein when the coupler is connected with the driver, the first coupling terminal and the second coupling terminal are coupled to each other and form an electrical connection channel.

14. The interventional medical device according to claim 12, further comprising:
a driving shaft, rotatably provided inside the catheter, wherein a distal end of the driving shaft is drivingly connected to the impeller;
a driving member, driven by the motor; and
a driven member, provided inside the coupler, and drivingly connected to a proximal end of the driving shaft,
wherein one of the driving member and the driven member is a magnetic component, and an other of the driving member and the driven member is a magnetic component or a conductor; in a state where the coupler is connected with the driver, the driven member and the driving member are spaced apart from and coupled to each other, thereby achieving power transmission from the driving member to the driven member; the catheter extends into an interior of the coupler and is in fluid communication with the second chamber; the driven member is located inside the second chamber and the proximal end of the driving shaft extends out from the proximal end of the catheter, enters an interior of the second chamber and is drivingly connected to the driven member.

15. The interventional medical device according to claim 12, wherein the interventional sheath comprises:
an interventional sheath catheter;
a sheath tube base, formed at a proximal end of the interventional sheath catheter, wherein the catheter is inserted from a proximal end of the sheath tube base, passes through the interventional sheath catheter, and exits from a distal end of the sheath tube base; and
a side branch tube, provided on the sheath tube base and in fluid communication with an internal channel of the interventional sheath catheter, wherein an end of the side branch tube is connected with a interface of the sheath tube base to communicate with the gap between the catheter and the interventional sheath catheter, and an other end of the side branch tube is in communication with the first chamber.

16. The interventional medical device according to claim 15, wherein the sheath tube base is provided with a suture buckle to achieve fixation through the suture buckle.

17. The interventional medical device according to claim 15, wherein the coupler is provided with the first chamber, a first joint and a second joint, the first joint and the second joint are both connected with the first chamber, the first joint being in communication with a first liquid storage bag of the first tubing assembly, and the second joint being in communication with the side branch tube.
